# EUROPÄISCHE PATENTANMELDUNG

(11) **EP 1 774 900 A1**
(43) Veröffentlichungstag der Anmeldung: **18.04.2007**
(21) Anmeldenummer: 06020914.5
(22) Anmeldetag: 05.10.2006
(51) Int. Cl.: A61B 3/135, G02B 26/08, G02B 26/10, G02B 21/06, G02B 21/00, A61B 3/14

(54) **Abbildendes optisches Gerät**

(30) Priorität: 14.10.2005 AT 16872005
(71) Anmelder: STEINHUBER, Wolfdietrich, 6080 Igls (AT)
(72) Erfinder: STEINHUBER, Wolfdietrich, 6080 Igls (AT)
(74) Vertreter: Torggler, Paul Norbert

(57) **Zusammenfassung**

Abbildendes optisches Gerät, insbesondere Ophthalmoskop oder Mikroskop oder Durchlichtmikroskop, mit einer Lichtquellenanordnung (20) und einem zumindest durch eine Beleuchtungslinsenanordnung (4) vorgegebenen Beleuchtungsstrahlengang (3) und einem zumindest durch eine Beobachtungslinsenanordnung (12) vorgegebenen Beobachtungsstrahlengang (11), wobei im Beleuchtungsstrahlengang (3) zumindest eine Beleuchtungsfeldblende (2) und im Beobachtungsstrahlengang (11) zumindest eine Beobachtungsfeldblende (13) angeordnet ist und der Beleuchtungsstrahlengang (3) und der Beobachtungsstrahlengang (11) in einem von 0° verschiedenen Winkel in einer Beleuchtungsbildebene (6), in die die Beleuchtungsfeldblende (2) von der Beleuchtungslinsenanordnung (4) abbildbar ist, angeordnet sind, wobei Beleuchtungslinsenanordnung (4) und Beobachtungslinsenanordnung (12) voneinander getrennte Linsenanordnungen sind, wobei ein einziger Licht reflektierender und um eine Schwenkachse (15) oszillierend hin und her schwenkbarer Spiegelkörper (14) im Beleuchtungsstrahlengang (3) und im Beobachtungsstrahlengang (11) angeordnet ist.

## Beschreibung

Die vorliegende Erfindung betrifft ein abbildendes optisches Gerät, insbesondere Ophthalmoskop oder Mikroskop oder Durchlichtmikroskop, mit einer Lichtquellenanordnung und einem zumindest durch eine Beleuchtungslinsenanordnung vorgegebenen Beleuchtungsstrahlengang und einem zumindest durch eine Beobachtungslinsenanordnung vorgegebenen Beobachtungsstrahlengang, wobei im Beleuchtungsstrahlengang zumindest eine Beleuchtungsfeldblende und im Beobachtungsstrahlengang zumindest eine Beobachtungsfeldblende angeordnet ist und der Beleuchtungsstrahlengang und der Beobachtungsstrahlengang in einem von 0° verschiedenen Winkel in einer Beleuchtungsbildebene, in die die Beleuchtungsfeldblende von der Beleuchtungslinsenanordnung abbildbar ist, angeordnet sind, wobei Beleuchtungslinsenanordnung und Beobachtungslinsenanordnung voneinander getrennte Linsenanordnungen sind.

Gattungsgemäße optische Geräte sind beim Stand der Technik zum Beispiel aus der WO 97/15855 und der DE 10050358 bekannt. ln diesen Schriften sind in den gezeigten Ausführungsbeispielen sowohl im Beobachtungs- als auch im Beleuchtungsstrahlengang Feldblenden zur Begrenzung des Beobachtungs- bzw. Beleuchtungsstrahlenbildes angeordnet. Zur Erzielung einer optimalen Abbildung ist hierbei nötig, dass sowohl die Öffnung der Beobachtungsfeldblende als auch die der Beleuchtungsfeldblende bezüglich der jeweiligen Linsenanordnung in der Bildebene des abzubildenden Gegenstandes angeordnet sind. Durch ein gemeinsames Oszillieren der beiden Feldblendenöffnungen wird der zu beobachtende Gegenstand, vorzugsweise streifenweise, optisch abgetastet. Bei entsprechend schneller Bewegung der Feldblenden ergibt sich ein letztendlich für das menschliche Auge aus den einzelnen abgetasteten Teilbereichen bzw. Streifen zusammengesetztes Bild. Um die relativen Abstände zwischen den beiden Feldblendenöffnungen während des Oszillierens konstant zu halten, ist es allgemein üblich, die Beobachtungs- und die Beleuchtungsfeldblende in einer Baueinheit auszuführen.

Die US 2004/0174498 A1 zeigt ein Ophtalmoskop, bei dem im Beobachtungsstrahlengang und im Beleuchtungsstrahlengang jeweils eine oszillierende Feldblende vorgesehen ist. Die beiden Feldblenden sind miteinander gekoppelt und in einem Bereich angeordnet, in dem der Beleuchtungsstrahlengang und der Beobachtungsstrahlengang im Wesentlichen parallel zueinander verlaufen. In diesem Bereich sind auch voneinander getrennte Optiken im Beleuchtungs- und Beobachtungsstrahlengang angeordnet. Damit sich Beobachtungs- und Beleuchtungsstrahlengang in einer Bildebene schneiden können, ist eine gemeinsame Frontlinse für die beiden Strahlengänge vorgesehen.

Nachteil des Standes der Technik ist es, dass es beim Oszillieren der Feldblenden, insbesondere durch die gewünschte schnelle Bewegung, zu nicht zu vernachlässigen Trägheitskräften kommt, welche wiederum das gesamte Gerät in Schwingungen versetzen, wodurch letztendlich die Qualität des erzeugten Bildes leiden kann.

Aufgabe der Erfindung ist es daher, eine Alternative für das, vorzugsweise streifenweise, optische Abtasten des abzubildenden Gegenstandes zu schaffen, bei der das Gerät weniger in Schwingung versetzt wird.

Dies wird erfindungsgemäß erreicht, indem ein einziger, Licht reflektierender und um eine Schwenkachse oszillierend hin und her schwenkbarer, Spiegelkörper im Beleuchtungsstrahlengang und im Beobachtungsstrahlengang angeordnet ist.

Durch den oszillierend um eine Schwenkachse hin und her schwenkbaren Spiegelkörper kann der abzubildende Gegenstand bzw. ein Zwischenbild von ihm abgetastet werden. Es treten dabei bei der Oszillation des Spiegelkörpers nur sehr geringe Trägheitskräfte auf, sodass das erfindungsgemäße Gerät beim Betrieb weniger bzw. nicht spürbar in Schwingung versetzt wird. Dies steigert zusätzlich zur gattungsgemäßen schrägen Beleuchtung und der Verwendung von voneinander getrennten Beleuchtungs- und Beobachtungslinsenanordnungen insgesamt die Qualität der Abbildungen. Durch die Verwendung nur eines Spiegelkörpers für Beleuchtungs- und Beobachtungsstrahlengang ist automatisch eine Synchronisation erreicht. Darüber hinaus ist es möglich, auf eine Bewegung der Beobachtungs- und der Beleuchtungsfeldblende zu verzichten. Diese können also starr ausgebildet werden, womit die durch sie beim Stand der Technik hervorgerufenen Schwingungen vermieden sind.

Eine besonders günstige Lösung sieht vor, dass der oszillierend hin und her schwingende Spiegelkörper Teil eines Spiegelgalvanometers ist, der Schwenkantrieb des Spiegelkörpers also nach dem Prinzip eines Galvanometers arbeitet.

Weitere Einzelheiten und Merkmale der vorliegenden Erfindung ergeben sich aus der Figurenbeschreibung. Dabei zeigen:
- Fig. 1:: Eine schematische Darstellung eines Ophthalmoskopes gemäß des Standes der Technik,
- Fig. 2:: eine schematische Darstellung eines ersten erfindungsgemäßen Ausführungsbeispiels in einer Seitenansicht,
- Fig. 3:: eine Detaildarstellung aus Fig. 2 in einer Draufsicht auf den Spiegelkörper,
- Fig. 4:: eine Detaildarstellung zum Spiegelkörper gemäß Fig. 2 in einer Seitenansicht,
- Fig. 5:: eine schematische Darstellung eines zweiten erfindungsgemäßen Ausführungsbeispiels in einer Seitenansicht,
- Fig. 6:: eine Detaildarstellung aus Fig. 5 in einer Draufsicht auf den Spiegelkörper und
- Fig. 7:: eine schematische Darstellung eines dritten erfindungsgemäßen Ausführungsbeispiels.

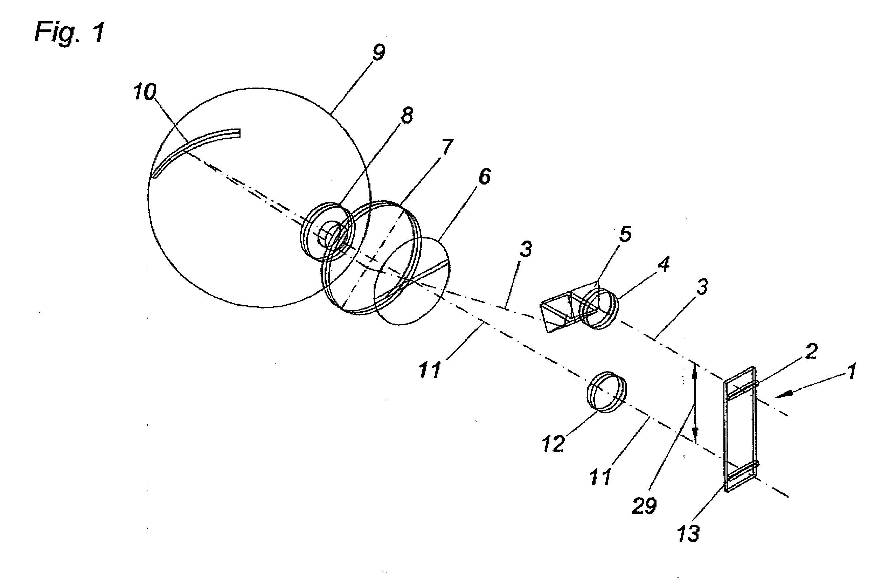

Bei dem in Fig. 1 dargestellten Stand der Technik wird die Retina 10 des Auges 9 als abzubildender Gegenstand mit Hilfe eines Ophthalmoskopes strichweise ausgeleuchtet bzw. abgetastet. Durch die Öffnung der Beleuchtungsfeldblende 2 im Beleuchtungsstrahlengang 3 tritt Licht einer nicht dargestellten Lichtquelle entlang des Beleuchtungsstrahlenganges 3 in das Beleuchtungsobjektiv (Beleuchtungslinsenanordnung) 4 und wird im gesteuerten und vorzugsweise verstellbaren Prismensystem 5 derart abgelenkt, dass der Beleuchtungsstrahlengang 3 in der Zwischenbildebene 6 mit dem Beobachtungsstrahlengang 11 in einem von 0° verschiedenen Winkel zusammenfällt, Man spricht hierbei von schräger Beleuchtung. Dabei ist die Zwischenbildebene 6 die Beleuchtungsbildebene, in die die Beleuchtungsfeldblerrde 2 von der Beleuchtungslinsenanordnung 4 abgebildet wird. Im Anschluss an die annähernde Parallelstellung des eintretenden Lichtbündels in der Ophthalmoskopielupe 7 tritt der Lichtstrahl durch die Pupille 8 in das Auge 9 ein und beleuchtet die dargestellten Lichtstreifen der Retina 10. Das dort gebeugte Licht wird entlang des Beobachtungsstrahlenganges 11 zurückgeworfen und kann nach Durchlaufen des Beobachtungsobjektives (Beobachtungslinsenanordnung) 12 und der Beobachtungsfeldblende 13 mit einem optischen Sensor (z.B. Auge eines Arztes) aufgenommen werden. Durch das Oszillieren der Doppelblende 1 wird der Lichtstreifen 10 auf der Retina verschoben. In der Doppelblende 1 sind hierbei die Beleuchtungsfeldblende 2 und die Beobachtungsfeldblende 13 in einer Baueinheit zusammengefasst. Oszilliert nun die Doppelblende 1 in der Bewegungsrichtung 29 mit einer entsprechend hohen Frequenz (z. B. 50 Hertz), so ergibt sich für ein das Licht aus dem Beobachtungsstrahlengang 11 in der Öffnung der Beobachtungsfeldblende 13 aufnehmendes menschliches Auge (oder einen entsprechenden Sensor) ein zusammenhängendes Abbild der Retina 10 des beobachteten Auges 9.

Der abzubildende Gegenstand sowie die Beleuchtungslinsenanordnung 4 und die Beleuchtungsfeldblende 2 sind hierbei in der Weise angeordnet, dass die Beleuchtungsfeldblende 2 über die Linsenanordnung 4 in die Beleuchtungsbildebene 6 und damit auf den abzubildenden Gegenstand abgebildet wird. Hierdurch erfolgt eine gezielte und bereichsweise, der Form der Öffnung der Beleuchtungsfeldblende 2 entsprechende Ausleuchtung des abzubildenden Gegenstandes. Dabei ist es unerheblich, ob der abzubildende Gegenstand sich direkt in der Beleuchtungsbildebene 6 befindet oder ob der abzubildende Gegenstand wie in Fig.1 in Form eines reellen Zwischenbildes in der Beleuchtungsbildebene 6 vorliegt. Die Abbildung des Gegenstandes in die Beleuchtungsbildebene 6 kann, z.B. wie in Fig. 1 gezeigt, mittels einer Ophthalmoskopielupe 7 erfolgen.

Der wie oben geschildert beleuchtete abzubildende Gegenstand wird über den Beobachtungsstrahlengang 11 und die Beobachtungslinsenanordnung 12 (hier wieder als einfache Sammellinse ausgebildet) auf der Beobachtungsbildebene abgebildet. Beim Stand der Technik ist es hierbei bekannt, in der Beobachtungsbildebene eine Beobachtungsfeldblende 13 anzuordnen. Dies ist vorzugsweise auch bei Geräten gemäß der Erfindung einzuhalten.

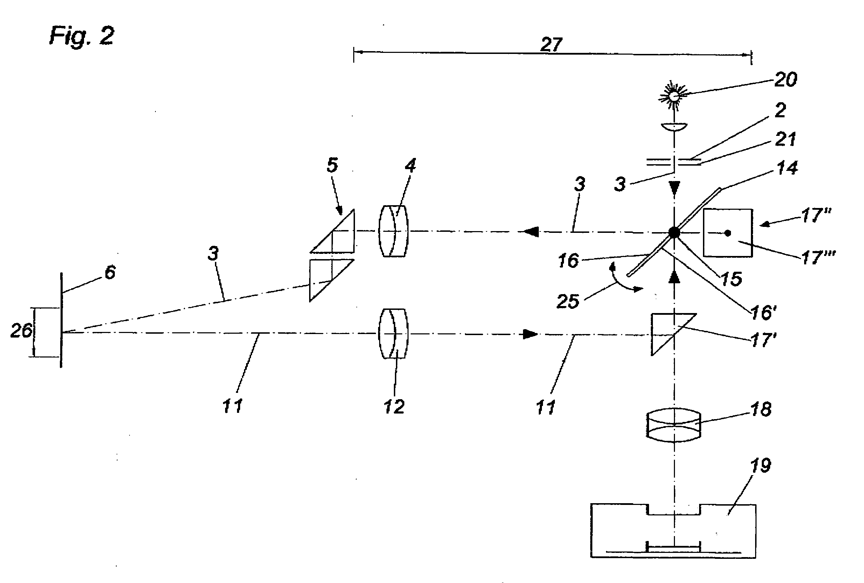

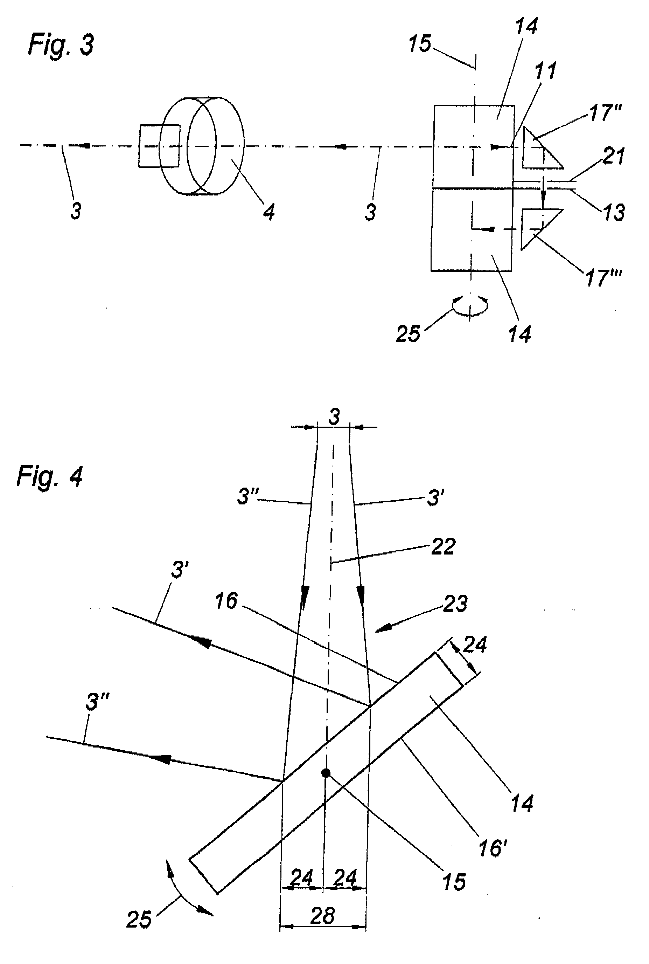

Fig. 2 zeigt nun ein erfindungsgemäßes Ausführungsbeispiel eines Ophthalmoskops. Wie auch beim Stand der Technik sorgen von einander getrennte Linsenanordnungen 4 und 12 im Beleuchtungsstrahlengang 3 und Beobachtungsstrahlengang 11 zusammen mit dem Prismensystem 5 dafür, dass Beleuchtungsstrahlengang 3 und Beobachtungsstrahlengang 11 in einem von 0° verschiedenen Winkel in der Beleuchtungsbildebene 6 aufeinander treffen. Der abzubildende Gegenstand kann direkt in der Beleuchtungsbildebene 6 angeordnet sein, womit die Beleuchtungsbildebene 6 dann im Wesentlichen in der Gegenstandsebene liegt. Diese Art der Anordnung ist vor allem bei erfindungsgemäßen Mikroskopen (Auflicht- oder Durchlichtmikroskop) günstig. Alternativ kann - vor allem bei Ophtalmoskopen - in der Beleuchtungsbildebene 6 aber auch ein reelles Zwischenbild des zu beobachtenden Gegenstandes, zum Beispiel der Retina 10, wie in Fig. 1 gezeigt, erzeugt werden. Die Beleuchtungsfeldblende 2 sowie die in der Draufsicht in Fig. 3 zu sehende Beobachtungsfeldblende 13 sind bevorzugt starr, also nicht beweglich - in Sinne von ortsfest - ausgeführt, wodurch die unerwünschten Schwingungen im Ophthalmoskop, wie sie beim Stand der Technik aufgrund der Bewegung der Feldblenden 2 und 13 auftreten, verhindert sind. Zur Beleuchtung ist die Lichtquellenanordnung 20 vorgesehen. Diese weist günstiger Weise ein Blitzlicht auf. Der nun erfindungsgemäß vorhandene, um die Schwenkachse 15 oszillierend in Richtung der Pfeile 25 hin und her schwenkbare Spiegelkörper 14 sorgt durch seine oszillierende Bewegung für ein Abtasten des zu beobachtenden Gegenstandes bzw. der Beleuchtungsbildebene 6.

Sowohl die Beleuchtungsfeldblende 2 als auch die Beobachtungsfeldblende 13 sind vorzugsweise schlitzförmig ausgebildet, was darin resultiert, dass die Beleuchtungsbildebene 6 streifenförmig abgetastet wird. Um eine durch die schräge Beleuchtung eventuell auftretende Ungleichverteilung der Lichtintensität entlang des beleuchteten Streifens zu kompensieren, kann vorgesehen sein, dass die Schlitze in den Feldblenden 2 und 13 V-förmig ausgebildet sind. Bei diesem und den in Folge gezeigten erfindungsgemäßen Ausführungsbeispielen können die Beleuchtungsfeldblende 2 und/oder die Beobachtungsfeldblende 13 wie auch gegebenenfalls vorhandene Raumfilterblenden 21 in ihrer Öffnungsweite verstellbar ausgeführt sein. Günstig ist es dabei, wenn die genannten Feldblenden 2 und/oder 13 und/oder 21 miteinander gekoppelt verstellbar sind, wodurch die Verstellung der einzelnen Feldblenden automatisch aufeinander abgestimmt ist.

Beim gezeigten Ausführungsbeispiel trifft das Licht auf dem Weg von der Lichtquellenanordnung 20 zum Flächenscanner 19 insgesamt 3 Mal auf den Spiegelkörper 14. Die erste Reflexion findet dabei im Beleuchtungsstrahlengang 3 an der oberen reflektierenden Oberfläche 16 des Spiegelkörpers 14 auf dem Weg von der Beleuchtungsfeldblende 2 zur Beleuchtungslinsenanordnung 4 statt. Im Beobachtungsstrahlengang 11 wird das Licht anschließend zunächst mit dem Umlenkprisma 17' auf die untere reflektierende Oberfläche 16' des Spiegelkörpers 14 gelenkt, um von dort auf das Umlenkprisma 17" (siehe Fig. 3) zu treffen. Dort wird das Licht wiederum reflektiert, um anschließend durch die Beobachtungsfeldblende 13 hindurch, zu einem dritten Umlenkprisma 17"' zu gelangen. In diesem wird das Licht wieder auf die untere reflektierende Oberfläche 16' des Spiegelkörpers 14 gelenkt. Dort erfolgt die Reflexion in Richtung der dem Flächenscanner 19 vorgeschalteten Linsenanordnung 18. Die Linsensanordnung 18 ist dabei so ausgelegt, dass das in der Ebene der Beobachtungsfeldblende 13 entstehende reelle Zwischenbild in die Aufnahmeebene des Flächenscanners 19 als reelles Bild abgebildet wird. Alle optischen Bauteile bis auf den Spiegelkörper 14 können dabei starr ausgebildet sein. Das Abtasten der Beleuchtungsbildebene erfolgt ausschließlich über die oszillierende Schwenkbewegung des Spiegelkörpers 14.

Durch die Verwendung eines einzigen Spiegelkörpers für Beleuchtungsstrahlengang 3 und Beobachtungsstrahlengang 11 ist automatisch die benötigte Synchronisation für eine korrekte Abbildung der abzubildenden Fläche 26 (in der Beleuchtungsbildebene 6) auf dem Flächenscanner 19 gegeben.

Die Länge, des optischen Strahlenweges zwischen Beleuchtungsfeldblende 2 und der das Licht des Beleuchtungsstrahlenganges 3 reflektierenden Oberfläche 16 des Spiegelkörpers 14 entspricht günstiger Weise der Länge des optischen Strahlenweges zwischen der das Licht des Beobachtungsstrahlenganges 11 reflektierenden Oberfläche 16' des Spiegelkörpers 14 und der Beobachtungsfeldblende 13. Dieselbe Länge sollte auch der optische Strahlenweg zwischen Beobachtungsfeldblende 13 und der reflektierenden Oberfläche 16' durch das Umlenkprisma 17"' hindurch haben. Die Umlenkprismen 17', 17" und 17"' könnten natürlich auch durch entsprechende Spiegel ersetzt werden.

Bei abbildenden optischen Geräten kommt es durch fehlerhafte Abbildungen von höheren Beugungsordnungen manchmal zu Verfälschungen des endgültigen Bildes. Um dies zu vermeiden, können außerhalb der Bildebenen angeordnete Raumfilter - zum Beispiel in Form von weiteren Blenden 21 mit schlitzförmigen Durchlassöffnungen - vorgesehen sein, wie dies beim Stand der Technik an sich bekannt ist. Durch die Anordnung der Blenden 21 außerhalb der Bildebenen werden höhere Beugungsordnungen ausgeblendet, sodass durch sie keine Abbildungsfehler entstehen können. Die oder der Raumfilter sind vorzugsweise ortsfest im abbildenden optischen Gerät angeordnet.

Um die abzubildende Fläche 26 möglichst gleichmäßig auszuleuchten und entsprechend gleichmäßig ausgeleuchtet abzubilden, ist günstiger Weise vorzusehen, dass der hier nicht explizit dargestellte Schwenkantrieb für den Spiegelkörper 14 es erlaubt, den Spiegelkörper 14 in der Art oszillierend hin- und her zu schwenken, dass das Licht des Beleuchtungsstrahlenganges 3 eine abzubildende Fläche in der Beleuchtungsbildebene 6 mit im Wesentlichen konstanter Geschwindigkeit überstreicht. Der Schwenkantrieb kann direkt oder über ein Getriebe an der Schwenkachse 15 angreifen. Eine günstige Variante sieht vor, dass als Schwenkantrieb ein Galvanometer verwendet wird.

Wenn gemäß einem bevorzugten Ausfiihrungsbeispiel die Lichtquellenanordnung eine Blitzlichtquelle aufweist, ist für eine entsprechende Synchronisation zwischen Lichtquelle 20, Schwenkbewegung des Spiegelkörpers 14 und Aufzeichnung durch den Flächenscanner 19 zu sorgen. Dies kann erreicht werden, indem die Blitzlichtquelle und der Schwenkantrieb des Spiegelkörpers 14 und der Flächenscanner 19 in der Art auf einander abgestimmt sind, dass während der Dauer eines Blitzes der Blitzlichtquelle der Spiegelkörper 14 zumindest ein Mal so weit schwenkbar ist, dass die abzubildende Fläche 26 in der Beleuchtungsbildebene 6 ein Mal vollständig überstreichbar ist und dass der Flächenscanner 19 das auf ihn auftreffende Bild vollständig aufnimmt.

Zu Fig. 3 sei noch bemerkt, dass es sich hierbei um eine Draufsicht auf den Ausschnitt 27 aus Fig. 2 handelt. Dabei sind die Lichtquellenanordnung 20, die Beleuchtungsfeldblende 2 und der darunter angeordnete Raumfilter 21 nicht dargestellt, um einen direkten Blick auf den Spiegelkörper 14, die Umlenkprismen 17" und 17"' und die Beobachtungsfeldblende 13 und die Raumfilterblende 21 zu ermöglichen. Betrachtet man Fig. 3, so trifft das von der Lichtquellenanordnung 20 bzw. Beleuchtungsfeldblende 2 kommende Licht senkrecht von oben auf die obere reflektierende Fläche 16 des Spiegelkörpers 14 und wird dann in Richtung des Beleuchtungsstrahlenganges 3 reflektiert. Auf dem Rückweg entlang des Beobachtungsstrahlenganges 11 trifft das Licht von unten auf die reflektierende Oberfläche 16' des Spiegelkörpers 14 und wird in Richtung des Ablenkprismas 17" reflektiert, um dann den bereits beschriebenen Weg durch die Beobachtungsfeldblende 13 zurück zur unteren Oberfläche 16' des Spiegelkörpers 14 zurückzulegen. Dort erfolgt die Reflektion in Richtung der Linsenanordnung 18. Der unterhalb des Spiegelkörpers 14 sich erstreckende und eigentlich in der Draufsicht nicht sichtbare Strahlenverlauf ist gestrichelt dargestellt.

Darüber hinaus ist Fig. 3 zu entnehmen, dass der Strahlenverlauf im Beobachtungsstrahlengang 11 vom ersten Ablenkprisma 17' zum Spiegelkörper 14 gegenüber dem Strahlenverlauf vom Spiegelkörper 14 zur Linsenanordnung 18 seitlich versetzt ist.

In Fig. 4 ist noch einmal vergrößert ein von der Beleuchtungsfeldblende 2 kommendes Lichtbündel 23 dargestellt. Zu sehen sind neben der gestrichelt dargestellten Mitte 22 des Lichtbündels 23 die Randstrahlen 3' und 3". Das Lichtbündel trifft dabei günstiger Weise in der Art auf den Spiegelkörper 14, dass die Mitte 22 im Wesentlichen im Bereich der Schwenkachse 15 auf dem Spiegelkörper 14 trifft. Hiervon ist in der Regel auszugehen, wenn die Mitte 22 des erzeugten Lichtbündels 23 nicht weiter von der Schwenkachse 15 entfernt auf den Spiegelkörper 14 trifft, als der kleinste Abstand 24 zwischen den Licht reflektierenden Oberflächen 16 und 16' des Spiegelkörpers 14 beträgt. In Fig. 4 ist dieser Bereich 28 explizit dargestellt. Darüber hinaus zeigt Fig. 4 auch, dass bei einem bevorzugten Ausführungsbeispiel des Spiegelkörpers 14 vorgesehen ist, dass dieser genau zwei ebene Licht reflektierende Oberflächen 16 und 16' auf zwei gegenüberliegenden Außenflächen aufweist. Die Licht reflektierenden Oberflächen 16 und 16' sind bevorzugt parallel zueinander angeordnet.

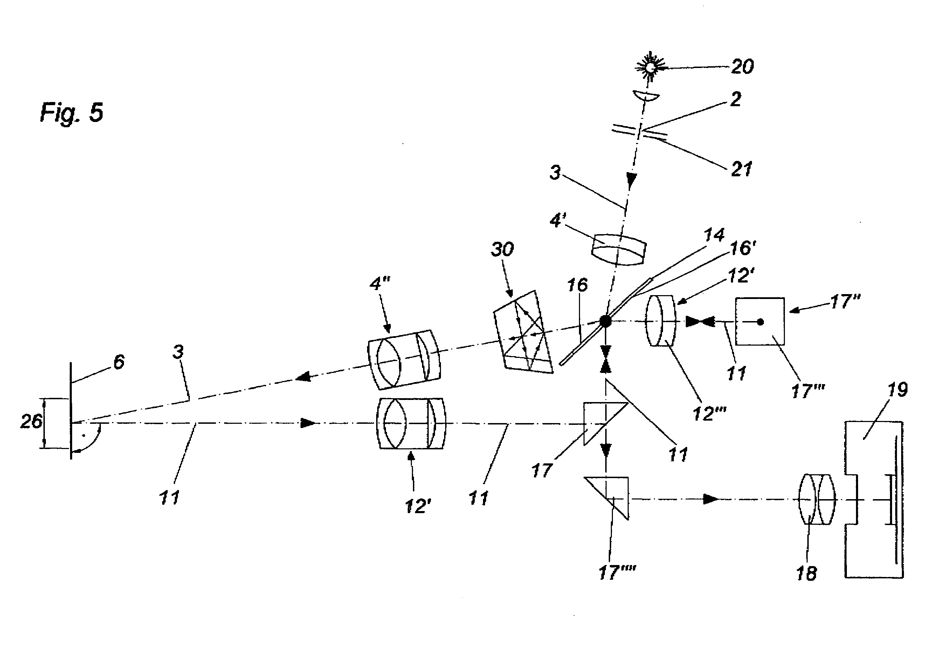

Fig. 5 zeigt ein zweites erfindungsgemäßen Ausführungsbeispiel in Form eines Ophtalmoskops, das in seinem Grundaufbau ähnlich dem in Fig. 2 gezeigten ist. Auch hier trifft der Beleuchtungsstrahlengang 3 in einem von 90° abweichenden Winkel auf, die abzubildende Fläche 26 bzw. die Beleuchtungsbildebene 6 während der Beobachtungsstrahlengang 11 im Wesentlichen senkrecht, also als Normale auf die abzubildende Fläche 26 verläuft. Diese Anordnung des Beobachtungsstrahlengangs 11 garantiert eine optimale Tiefenschärfe bei der Abbildung der abzubildenden Fläche 26. Bei einer von 90° abweichenden Anordnung zwischen abzubildender Fläche 26 bzw. Gegenstandsebene und Beobachtungsstrahlengang 11 wäre diese Tiefenschärfe nicht über die gesamte Fläche 26 gegeben.

Im Gegensatz zu Fig. 2 ist in dem Ausführungsbeispiel gemäß Fig. 5 ein Umkehrprisma 30 im Beleuchtungsstrahlengang 3 anstelle der Prismen 5 angeordnet. Umkehrsysteme sind an sich beim Stand der Technik bekannt und dienen der Bildumkehr um 180°. Als Umkehrsystem 30 können zum Beispiel beim Stand der Technik bekannte Pechan-Prismen mit Dachkante oder sogenannte Porrosysteme eingesetzt werden. Das Umkehrsystem 30 kann anstelle seiner Anordnung im Beleuchtungsstrahlengang 3 auch im Beobachtungsstrahlengang 11 angeordnet sein.

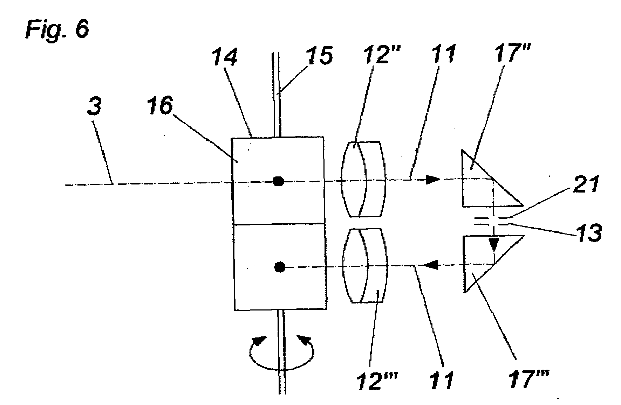

Ein weiterer Unterschied zu Fig. 2 besteht darin, dass sowohl die Beleuchtungslinsenanordnung 4 als auch die Beobachtungslinsenanordnung 12 jeweils mindestens 2 voneinander räumlich getrennte Linsen 4' und 4" sowie 12' und 12" aufweisen. Die Linsen 4' bzw. 12' erzeugen im Beleuchtungsstrahlengang 3 bzw. im Beobachtungsstrahlengang 11 parallele Strahlen, während die Linsen 4" und 12" diese parallelen Strahlen wiederum in konvergente Strahlenbündel verwandeln. In der in Fig. 5 gewählten seitlichen Darstellung ist die Linse 12" von der Linse 12"' verdeckt und daher nicht direkt zu sehen. Es wird diesbezüglich auf die weiter unten noch einmal kurz erläuterte Fig. 6. verwiesen. Die Linse 12'" erzeugt wiederum parallele Strahlenbündel, die durch die Linsenanordnung 18 wieder zu einem konvergenten Strahlenbündel mit Brennpunkt auf dem Flächenscanner 19 verwandelt werden. Durch diese Anordnung der Linsen im Beleuchtungs-3 und Beobachtungsstrahlengang 11 ist sichergestellt, dass im Bereich des Spiegelkörpers 14 immer parallele Strahlen vorhanden sind, was die Abbildungsqualität insgesamt steigert. Daher ist es günstig, wenn der Spiegelkörper 14 im Bereich der parallelen Strahlen zwischen den getrennten Linsen 4', 4", 12', 12", 12"' und 18 angeordnet ist.

Fig. 6 zeigt in einer Draufsicht die Anordnung der Beobachtungsfeldblende 13 zwischen den beiden Umlenkprismen 17" und 17"'. Der Beleuchtungsstrahlengang 3 wird an der Oberseite 16 des Spiegelkörpers 14 reflektiert. Das Licht des Beobachtungsstrahlenganges 11 trifft von unten auf die gegenüberliegende reflektierende Oberfläche 16' des Spiegelkörpers 14 und wird durch die Linse 12" in ein konvergentes Strahlenbündel verwandelt, welches mittels des Umlenkprismas 17" durch die Raumfilterblende 21 hindurch in die im Bereich der Beobachtungsfeldblende 13 angeordnete Beobachtungsbildebene umgelenkt wird. Von dort aus verläuft der Beobachtungsstrahlengang 11 wieder in Richtung der unteren reflektierenden Fläche 16" des Spiegelkörpers 14 und wird in Richtung Umlenkprisma 17"" abgelenkt. Der weitere Strahlenverlauf in Richtung Flächenscanner 19 ist wiederum Fig. 5 zu entnehmen. Durch die gewählte Anordnung ist wie auch bei dem Ausführungsbeispiel in Fig. 2 gewährleistet, dass der Beleuchtungsstrahlengang 3, insbesondere zwischen der Lichtquellenanordnung 20 oder der Beleuchtungsfeldblende 2 und der Beleuchtungsbüdebene 6 vollständig getrennt vom Beobachtungsstrahlengang 11, vorzugsweise zwischen der Beleuchtungsbildebene 6 und der Beobachtungsfeldebene 13 oder einem optischen Sensor bzw. Flächenscanner 19 verläuft. Das gleiche gilt für die erste Teilstrecke des Beobachtungsstrahlenganges 11 zwischen der Beleuchtungsbildebene 6 und der Beobachtungsfeldblende 13 und der zweiten Teilstrecke des Beobachtungsstrahlenganges 11 zwischen der Beobachtungsfeldblende 13 und einem optischen Sensor bzw. Flächenscanner 19. Es liegen somit drei voneinander vollkommen getrennte Strahlengänge vor, wodurch ein besonders kontrastscharfes Abbild des zu untersuchenden Objektes erreicht wird. Unter vollständig getrennten Strahlengängen ist dabei insbesondere zu verstehen, dass sich die genannten Strahlengänge weder schneiden noch überlagern noch irgendeine gemeinsame Teilstrecke haben. Hierdurch werden die zum Beispiel zur Augenuntersuchung benötigten hohen Kontraste gewährleistet.

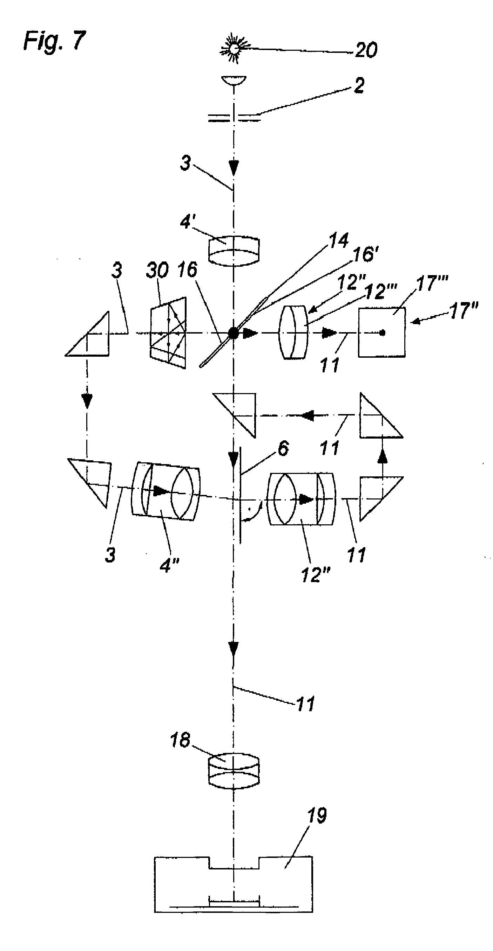

In Fig. 7 ist ein drittes erfindungsgemäßes Ausführungsbeispiel in Form eines schematisierten Durchlichtmikroskops gezeigt. Der Beobachtungsstrahlengang 3 trifft in einem von der normalen bzw. senkrechten abweichenden Winkel auf das in der Beleuchtungsbildebene bzw. Gegenstandsebene 6 angeordnete zu beobachtende bzw. zu durchleuchtende Objekt. Der Beobachtungsstrahlengang 11 ist hingegen wiederum in einer Normalen, also senkrecht auf die Beleuchtungsbildebene bzw. Gegenstandsebene 6 angeordnet. Auch in diesem Ausführungsbeispiel liegt also wieder ein von 0° abweichender Winkel zwischen Beleuchtungsstrahlengang 3 und Beobachtungsstrahlengang 11 vor. Wie in dem Ausführungsbeispiel gemäß Fig. 5 sind auch hier getrennte Linsen 4' und 4" für den Beleuchtungsstrahlengang sowie 12', 12", 12'" und 18 für den Beobachtungsstrahlengang 11 vorgesehen. Die Linse 12" ist wie in Fig. 6 dargestellt in der in Fig. 7 gewählten Seitendarstellung hinter der Linse 12'" verborgen. Auch hier liegen wiederum drei räumlich vollständig voneinander getrennte Strahlengänge vor. Der erste ist wiederum der Beleuchtungsstrahlengang 3 zwischen Lichtquellenanordnung 20 und Beleuchtungsbildebene 6. Der zweite Strahlengang ist die erste Teilstrecke des Beobachtungsstrahlenganges 11 zwischen Beleuchtungsbildebene 6 und Beobachtungsfeldblende 13. Der dritte Strahlengang ist die zweite Teilstrecke des Beobachtungsstrahlengangs 11 zwischen Beobachtungsfeldblende 13 und Flächenscanner 19. Auch bei dem Durchlichtmikroskop gemäß Fig. 7 kann bei Bedarf sowohl im Beleuchtungsstrahlengang 3 als auch im Beobachtungsstrahlengang 11 ein hier im Beleuchtungsstrahlengang 3 gezeigtes Umkehrsystem 30 angeordnet sein.

In allen drei erfindungsgemäßen Ausführungsbeispielen gemäß der Fig. 2 bis 7 trifft der Beleuchtungsstrahlengang 3 von oben auf die reflektierende Fläche 16 des oszillierenden Spiegelkörpers 14 während der Beobachtungsstrahlengang 11 zweifach, in Form von zwei räumlich getrennten Reflexionsbereichen von unten auf die untere reflektierende Oberfläche 16' des Spiegelkörpers 14 trifft.

Alternativ wäre es aber auch möglich, Ausführungsbeispiele so zu gestatten, dass alle drei Spiegelungen nur an der oberen Reflexionsfläche 16 oder nur an der unteren Reflexionsfläche 16' des Spiegelkörpers 14 stattfinden. Darüber hinaus sind auch Ausführungsbeispiele denkbar, bei denen für jede Spiegelung eine eigene Reflexionsoberfläche, also zum Beispiel Spiegelkörper 14 mit drei Reflexionsoberflächen vorgesehen sind. Auch andere Mischformen sind möglich, solange ein einziger schwenkbarer Spiegelkörper 14 im Beleuchtungsstrahlengang 3 und im Beobachtungsstrahlengang 11 angeordnet ist, was die eingangs erwähnte Synchronisation gewährleistet.

Die erfindungsgemäßen Geräte können bei entsprechender Auslegung mit Licht aus dem gesamten Lichtwellenlängenbereich betrieben werden. Bevorzugt handelt es sich um sichtbares Licht. Es sind aber auch andere Wellenlängenbereiche möglich. Um eine Rekonstruktion der einzelnen gescannten Abschnitte bzw. Streifen zu einem Gesamtbild sicherzustellen, ist es günstig, eine Oszillationsfrequenz des Spiegelkörpers 14 von mindesten 50 Hz zu wählen. Höhere Frequenzen führen zu einer weiteren Verbesserung des entstehenden Abbildes. Anstelle des vorzugsweise digitalen Flächenscanners 19 können natürlich auch andere Kameras und dergleichen eingesetzt werden. Natürlich ist es auch möglich, dass im Anschluss an die Linsenanordnung 18 entstehende Bild direkt zu betrachten. Die hier nicht in Form eines eigenen Ausführungsbeispiel gezeigten (Auflicht)mikroskope sind in den erfindungswesentlichen Punkten wie Ophtalmoskope ausführbar.

## Patentansprüche

1. Abbildendes optisches Gerät, insbesondere Ophthalmoskop oder Mikroskop oder Durchlichtmikroskop, mit einer Lichtquellenanordnung und einem zumindest durch eine Beleuchtungslinsenanordnung vorgegebenen Beleuchtungsstrahlengang und einem zumindest durch eine Beobachtungslinsenanordnung vorgegebenen Beobachtungsstrahlengang, wobei im Beleuchtungsstrahlengang zumindest eine Beleuchtungsfeldblende und im Beobachtungsstrahlengang zumindest eine Beobachtungsfeldblende angeordnet ist und der Beleuchtungsstrahlengang und der Beobachtungsstrahlengang in einem von 0° verschiedenen Winkel in einer Beleuchtungsbildebene, in die die Beleuchtungsfeldblende von der Beleuchtungslinsenanordnung abbildbar ist, angeordnet sind, wobei Beleuchtungslinsenanordnung und Beobachtungslinsenanordnung voneinander getrennte Linsenanordnungen sind, **dadurch gekennzeichnet, dass** ein einziger Licht reflektierender und um eine Schwenkachse (15) oszillierend hin und her schwenkbarer Spiegelkörper (14) im Beleuchtungsstrahlengang (3) und im Beobachtungsstrahlengang (11) angeordnet ist.

2. Abbildendes optisches Gerät nach Anspruch 1, **dadurch gekennzeichnet, dass** der Spiegelkörper (14), vorzugsweise genau, zwei ebene Licht reflektierende Oberflächen .(16, 16') auf zwei gegenüberliegenden Außenflächen aufweist.

3. Abbildendes optisches Gerät nach Anspruch 2, **dadurch gekennzeichnet, dass** die Licht reflektierenden Oberflächen (16, 16') parallel zueinander angeordnet sind.

4. Abbildendes optisches Gerät nach einem der Ansprüche 1 bis 3, **dadurch gekennzeichnet, dass** der Spiegelkörper (14) Teil eines Spiegelgalvanometers ist.

5. Abbildendes optisches Gerät nach einem der Ansprüche 1 bis 4, **dadurch gekennzeichnet, dass** der Spiegelkörper (14) bzw. seine Schwenkachse (15) derart angeordnet ist, dass im Beleuchtungsstrahlengang (3) die Mitte (22) eines mittels Lichtquellenanordnung (20) und Beleuchtungsfeldblende (2) erzeugten Lichtbündels (23) im Wesentlichen im Bereich der Schwenkachse (15) auf den Spiegelkörper (14) trifft.

6. Abbildendes optisches Gerät nach Anspruch 5, **dadurch gekennzeichnet, dass** die Mitte (22) des erzeugten Lichtbündels (23) nicht weiter von der Schwenkachse (15) entfernt auf den Spiegelkörper (14) trifft, als der kleinste Abstand (24) zwischen den Licht reflektierenden Oberflächen (16,16') des Spiegelkörpers (14) beträgt.

7. Abbildendes optisches Gerät nach einem der Ansprüche 1 bis 6, **dadurch gekennzeichnet, dass** die Beleuchtungsfeldblende (2) und/oder die Beobachtungsfeldblende (13) im Wesentlichen schlitzförmige Durchlassöffnungen für Licht aufweisen.

8. Abbildendes optisches Gerät nach Anspruch 7, **dadurch gekennzeichnet, dass** die schlitzförmigen Durchlassöffnungen V-förmig ausgebildet sind.

9. Abbildendes optisches Gerät nach einem der Ansprüche 1 bis 8, **dadurch gekennzeichnet, dass** das abbildende optische Gerät so ausgebildet ist, dass das Licht der Lichtquellenanordnung (20) auf dem Beleuchtungsstrahlengang (3) ein Mal am Spiegelkörper (14) reflektiert und auf dem Beobachtungsstrahlengang (11) zwei Mal am Spiegelkörper (14) reflektierbar ist.

10. Abbildendes optisches Gerät nach einem der Ansprüche 1 bis 9, **dadurch gekennzeichnet, dass** die Länge des optischen Strahlenweges zwischen Beleuchtungsfeldblende (2) und der das Licht des Beleuchtungsstrahlengangs (3) reflektierenden Oberfläche (16) des Spiegelkörpers (14) der Länge des optischen Strahlenweges zwischen der das Licht des Beobachtungsstrahlengangs (11) reflektierenden Oberfläche (1B') des Spiegelkörpers (14) und der Beobachtungsfeldblende (13) und der Länge des optischen Strahlenweges zwischen der Beobachtungsfeldblende (13) und der das Licht des Beleuchtungsstrahlengangs reflektierenden Oberfläche (16') des Spiegelkörpers (14) entspricht.

11. Abbildendes optisches Gerät nach einem der Ansprüche 1 bis 10, **dadurch gekennzeichnet, dass** zumindest ein Raumfilter, vorzugsweise in Form einer Blende (21) mit im Wesentlichen schlitzförmiger Durchlassöffnung, außerhalb der Bildebenen des abbildenden optischen Gerätes angeordnet ist.

12. Abbildendes optisches Gerät nach einem der Ansprüche 1 bis 11, **dadurch gekennzeichnet, dass** eine Linsenanordnung (18) zur Abbildung eines in der Beobachtungsfeldblende (13) entstehenden Bildes auf einen, vorzugsweise digitalen, Flächenscanner (19) vorgesehen ist.

13. Abbildendes optisches Gerät nach einem der Ansprüche 1 bis 12, **dadurch gekennzeichnet, dass** ein Schwenkantrieb für den Spiegelkörper (14) vorgesehen ist, der es erlaubt, den Spiegelkörper (14) in der Art oszillierend hin und her zu schwenken, dass das Licht des Beleuchtungsstrahlenganges (3) eine abzubildende Fläche (26) in der Beieuchtungsbitdebene (6) mit im Wesentlichen konstanter Geschwindigkeit überstreicht

14. Abbildendes optisches Gerät nach einem der Ansprüche 1 bis 13, **dadurch gekennzeichnet, dass** die Lichtquellenanordnung (20) eine Blitzlichtquelle aufweist.

15. Abbildendes optisches Gerät nach den Ansprüchen 12 und 13 und 14, **dadurch gekennzeichnet, dass** die Blitzlichtquelle und der Schwenkantrieb des Spiegelkörpers (14) und der Flächenscanner (19) in der Art synchronisiert sind, dass während der Dauer eines Blitzes der Blitzlichtquelle der Spiegelkörper (14) zumindest ein Mal so weit schwenkbar ist, dass die abzubildende Fläche (26) in der Beleuchtungsbildebene (6) ein Mal vollständig überstreichbar ist und dass der Flächenscanner (19) das auf ihn auftreffende Bild vollständig aufnimmt.

16. Abbildendes optisches Gerät nach einem der Ansprüche 1 bis 15, **dadurch gekennzeichnet, dass** ein erstes Umlenkprisma (17") oder ein erster Spiegel und ein zweites Umlenkprisma (17"') oder ein zweiter Spiegel so angeordnet sind, dass das Licht auf dem Beobachtungsstrahlengang (11) vom Spiegelkörper (14) kommend von dem ersten Umlenkprisma (17") oder dem ersten Spiegel zur Beobachtungsfeldblende (13) und weiter von dem zweiten Umlenkprisma (17"') oder dem zweiten Spiegel zum Spiegelkörper (14) hin reflektierbar ist.

17. Abbildendes optisches Gerät nach einem der Ansprüche 1 bis 16, **dadurch gekennzeichnet, dass** der Beobachtungsstrahlengang (11) im Wesentlichen senkrecht zur abzubildenden Fläche (26) bzw. als Normale auf die abzubildende Fläche (26) verläuft.

18. Abbildendes optisches Gerät nach einem der Ansprüche 1 bis 17, **dadurch gekennzeichnet, dass** der Beleuchtungsstrahlengang (3), vorzugsweise zwischen der Lichtquellenanordnung (20) oder der Beleuchtungsfeldblende (2) und der Beleuchtungsbildebene (6), vollständig getrennt vom Beobachtungsstrahlengang (11), vorzugsweise zwischen der Beleuchtungsbildebene (6) und der Beobachtungsfeldblende (13) oder einem optischen Sensor bzw. Flächenscanner (19), verläuft.

19. Abbildendes optisches Gerät nach einem der Ansprüche 1 bis 18, **dadurch gekennzeichnet, dass** eine erste Teilstrecke des Beobachtungsstrahlenganges (11) zwischen Beleuchtungsbildebene (6) und Beobachtungsfeldblende (13) vollständig getrennt von einer zweiten Teilstrecke des Beobachtungsstrahlenganges (11) zwischen der Beobachtungsfeldblende (13) und einem optischen Sensor bzw. Flächenscanner (19) verläuft.

20. Abbildendes optisches Gerät nach einem der Ansprüche 1 bis 19, **dadurch gekennzeichnet, dass** die Beleuchtungslinsenanordnung (4) und/oder die Beobachtungslinsenanordnung (12) jeweils mindestens zwei voneinander räumlich getrennte Linsen (4', 4", 12', 12") aufweisen, wobei zwischen den getrennten Linsen (4', 4") der Beleuchtungslinsenanordnung (2) und/oder den getrennten Linsen (12', 12") der Beobachtungslinsenanordnung (12) jeweils parallele Strahlen verlaufen.

21. Abbildendes optisches Gerät nach Anspruch 20, **dadurch gekennzeichnet, dass** der Spiegelkörper (14) im Bereich paralleler Strahlen zwischen den getrennten Linsen (4', 4") der Beleuchtungslinsenanordnung (4) und/oder zwischen den getrennten Linsen (12', 12") der Beobachtungslinsenanordnung (12) angeordnet ist.

22. Abbildendes optisches Gerät nach einem der Ansprüche 1 bis 21, **dadurch gekennzeichnet, dass** im Beleuchtungsstrahlengang (3) und/oder im Beobachtungsstrahlengang (11) ein Umkehrsystem (30) angeordnet ist.

23. Abbildendes optisches Gerät nach einem der Ansprüche 1 bis 22, **dadurch gekennzeichnet, dass** die Beleuchtungsfeldblende (2) und/oder die Beobachtungsfeldblende (13) und/oder gegebenenfalls vorhandene Raumfilterblenden (21), vorzugsweise miteinander gekoppelt, in ihrer Blendenöffnungsweite verstellbar sind.
